# FASCICULE DE BREVET EUROPEEN

(11) **EP 1 737 316 B1**
(45) Date de publication et mention de la délivrance du brevet: **12.09.2007**
(21) Numéro de dépôt: 05731110.2
(22) Date de dépôt: 23.02.2005
(51) Int. Cl.: A47F 1/08, B65D 83/00

(54) **DISTRIBUTEUR DE MASQUES FACIAUX**
GESICHTSMASKENABGABEVORRICHTUNG
FACE MASK DISTRIBUTOR

(30) Priorité: 23.02.2004 FR 0401767
(43) Date de publication de la demande: 03.01.2007
(73) Titulaire: Bacou Dalloz Plaintel, 22940 Plaintel (FR)
(72) Inventeur: MARGERIT, Pascal, 77750 Saint-Cyr-Sur-Morin (FR); DE ZAIACOMO, Olivier, 22150 Ploeuc sur Lie (FR); BARGEOT, Philippe, 22000 Saint-Brieuc (FR); GAUTIER, Joël, 22800 Quintin (FR)
(74) Mandataire: Texier, Christian
(86) Numéro de dépôt international: PCT/FR2005/000417
(87) Numéro de publication internationale: WO 2005/082205

(56) Documents cités:
- FR-A- 2 706 864
- US-A- 2 340 090
- US-A- 2 804 236
- US-A- 4 550 856

## Description

L'invention concerne un distributeur à suspendre, à sortie par le bas, apte à contenir à coulisse une pile verticale de masques faciaux dans une chambre séparée de cette sortie par une zone où la section droite de la chambre est rétrécie afin d'arrêter le masque inférieur de la pile, tout en permettant d'extraire à force ce masque par la section rétrécie.

La publication US 2 804 236 décrit un distributeur conçu pour la distribution de gobelets.

Il est constitué d'un film étirable et la section droite rétrécie est délimitée par deux soudures verticales opposées qui arrêtent le gobelet inférieur par sa collerette et qui peuvent s'écarter pour permettre le passage à force du gobelet dont la rigidité est suffisante pour provoquer l'étirement de la section rétrécie.

La publication US 2 340 090 décrit un autre distributeur de gobelets constitué par un sac flexible dont la sortie est rétrécie et pourvue d'un élastique de façon à serrer élastiquement et latéralement le dernier gobelet qui est partiellement en saillie à l'extérieur du distributeur, l'extraction du gobelet se faisant en tirant la partie en saillie du gobelet, ce qui force l'élastique à s'étirer.

La publication FR 2 706 864 décrit un distributeur en panneau de matière souple dont l'orifice de sortie coopère avec un élastique pour s'agrandir quand cela est nécessaire.

Ce distributeur n'est pas conçu pour contenir une pile d'objets mais des sacs serrés les uns contre les autres.

La présente invention concerne un distributeur conçu pour distribuer des masques faciaux relativement mous qui ne présentent pas une rigidité transversale comparable à celle des gobelets et qui ne pourraient être retenus par leur collerette ni passer à force au travers d'une section élastique.

Ces masques sont par exemple des coquilles à paroi latérale filtrante, conformées pour présenter une base qui détermine éventuellement une ouverture munie d'une soupape d'expiration et pour présenter à l'opposé de cette base une ouverture large bordée par une collerette et apte à s'appliquer sur le visage en couvrant la bouche et le nez, et entre la base et la collerette une paroi latérale sans raideur, l'ensemble du masque étant facilement déformable sous l'effet d'une sollicitation latérale et apte à reprendre sa forme au repos.

La publication US 4 550 856 décrit un distributeur de masques constitué par une boite parallélépipédique préformée en carton, ouvrante à sa base, laquelle est pourvue d'une structure rapportée qui détermine une section de passage rétrécie où le dernier masque de la pile est arrêté par une lèvre horizontale de la structure qui a sensiblement la conformation transversale du masque et qui laisse passer à l'extérieur du distributeur la majeure partie du masque.

Un tel distributeur est beaucoup plus coûteux à fabriquer que les distributeurs non rigides fabriqués en film, et la nécessité de fabriquer séparément la structure qui fournira la zone rétrécie et de rapporter cette structure dans le distributeur est aussi génératrice d'un surcoût élevé.

De plus, le fait qu'en service le masque prêt à être extrait soit en attente en majeure partie en saillie à l'extérieur du distributeur est défavorable pour l'hygiène, notamment dans l'environnement d'un atelier.

La présente invention a pour but de fournir un distributeur de masques faciaux peu coûteux à fabriquer, sans structure rapportée, sans élastique, où le masque inférieur de la pile reste contenu dans le distributeur en attente d'être extrait.

Le distributeur selon l'invention pour distribuer des masques faciaux à paroi latérale filtrante empilés avec leurs collerettes tournées vers le haut, ces masques étant déformables transversalement et conformés pour présenter un fond et, à l'opposé de ce fond, une ouverture large bordée par une collerette, et apte à s'appliquer sur le visage par la collerette en couvrant la bouche et le nez, et entre le fond et la collerette une paroi latérale filtrante sans raideur, ce distributeur étant du type à suspendre, à sortie par le bas, apte à contenir à coulisse une pile verticale des masques faciaux dans une chambre séparée de cette sortie par une zone où la section droite de la chambre est rétrécie afin d'arrêter le masque inférieur de la pile, tout en permettant d'extraire à force ce masque par la section rétrécie, ce distributeur étant caractérisé en ce qu'il est essentiellement constitué par une gaine tubulaire allongée déformable et non extensible, en film mince thermosoudable, qui forme deux parois en vis-à-vis solidarisées par leurs bords longitudinaux et maintenues localement écartées l'une de l'autre par la pile des masques contenue dans la gaine, les deux parois étant soudées l'une à l'autre sous la pile par deux soudures obliques en vis-à-vis qui partent des bords longitudinaux des deux parois en réduisant progressivement la section droite de la gaine jusqu'à une section rétrécie en sorte que le masque inférieur de la pile soit retenu entre les soudures obliques par contact latéral avec la gaine et que le fond de ce masque soit en saillie sous la section rétrécie, et la gaine présentant en dessous de la section rétrécie une sortie de gaine située au dessous du fond du masque inférieur, et fermée au moins provisoirement par une fermeture.

La sortie de la gaine peut être fermée par une ligne de soudure qui est éliminée pour la mise en service du distributeur. Elle peut aussi être fermée par une fermeture ouvrant à la demande, par exemple une fermeture à curseur, ou à boucles coopérantes, ou à contacts adhésifs.

On décrira ci-après un exemple de réalisation d'un distributeur selon l'invention, en référence aux figures du dessin joint sur lequel :
- la figure 1 représente les phases successives de la mise en place d'un masque (M) sur un visage ;
- la figure 2 est un schéma en plan d'un distributeur selon l'invention vidé et mis à plat ;
- la figure 3 est un schéma en plan analogue à celui de la figure 2 où le distributeur contient des plaquettes de renfort ;
- la figure 4 est un schéma partiel, en plan, analogue à celui de la figure 2 où le distributeur contient une pile de masques ;
- la figure 5 est une vue en perspective du distributeur rempli mais sans plaquette de renfort dans la poche, et
- la figure 6 est une coupe transversale de la poche du distributeur.

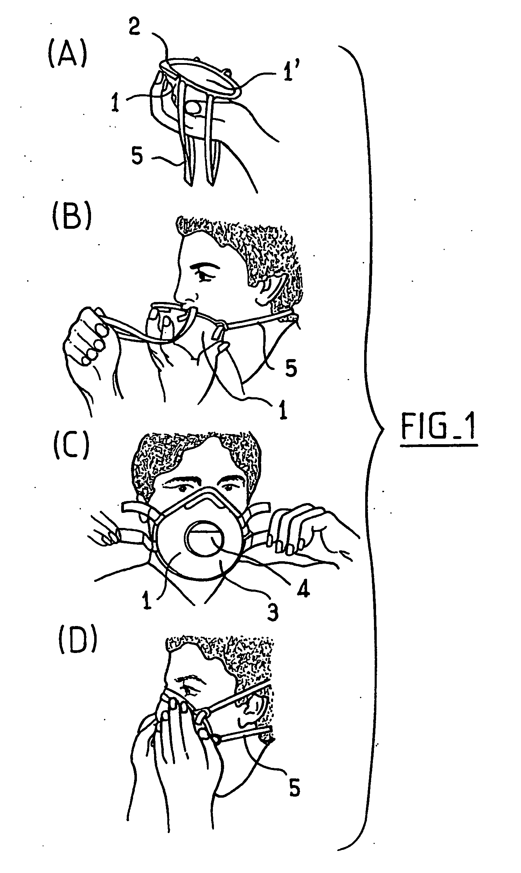

La figure 1 représente les phases successives de la mise en place d'un masque (M) qui est successivement pris en main (figure 1A), présenté au visage (figure 1B), appliqué de façon à couvrir la bouche et le nez (figure 1C) et maintenu en place par des brides élastiques (figure 1D), de façon en soi connue.

Le masque a la forme générale d'une coquille filtrante sans raideur constituée d'une double paroi (1,1') en non tissé (de polyester par exemple) qui emprisonne un média filtrant, par exemple en polypropylène, qui présente une collerette d'ouverture (2), un fond (3) éventuellement muni d'une soupape d'expiration (4), et des brides latérales (5).

Le distributeur est une gaine allongée à paroi mince en film transparent, par exemple en polyéthylène, d'une épaisseur de 30 à 200 micromètres, de préférence voisine de 40 micromètres mais qui pourrait approcher les 150 micromètres si désiré.

La gaine lorsqu'elle est vide et aplatie forme deux parois rectangulaires qui sont solidaires le long de leurs bords longitudinaux AB, CD et qui sont encore solidarisées par différentes soudures.

Les deux parois (F,F') peuvent provenir d'une même nappe pliée sur elle-même de façon à constituer les deux parois qui sont ensuite solidarisées localement par soudage, ou peuvent provenir de deux nappes appliquées l'une sur l'autre et soudées le long de leurs bords.

Outre leurs soudures longitudinales (6,6') le long des bords AB et CD, les deux parois sont soudées par une soudure transversale (7) et deux soudures obliques (8,8') qui déterminent entre elles et entre les soudures (6,6') une poche T apte à contenir une pile de masques et éventuellement une plaquette de renfort.

Les soudures obliques (8,8') s'étendent par exemple sur une hauteur de 50 millimètres.

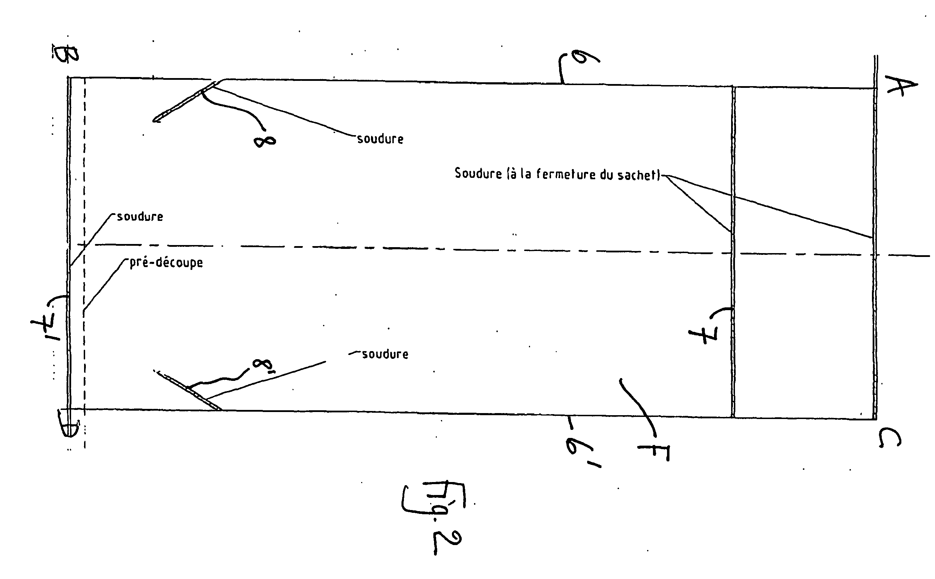

La figure 2 est une vue en plan de l'une des parois. Cette paroi (F), de forme générale rectangulaire est solidarisée de l'autre paroi (non visible sur la figure) contre laquelle elle est appliquée, par les soudures longitudinales (6,6'), par une soudure supérieure transversale et continue (7), par de courtes soudures obliques (8, 8') qui réduisent localement la section de la gaine sous la pile des masques (non représentée sur la figure 2), et par une soudure transversale inférïeure (7') qui scelle la gaine à distance des soudures obliques.

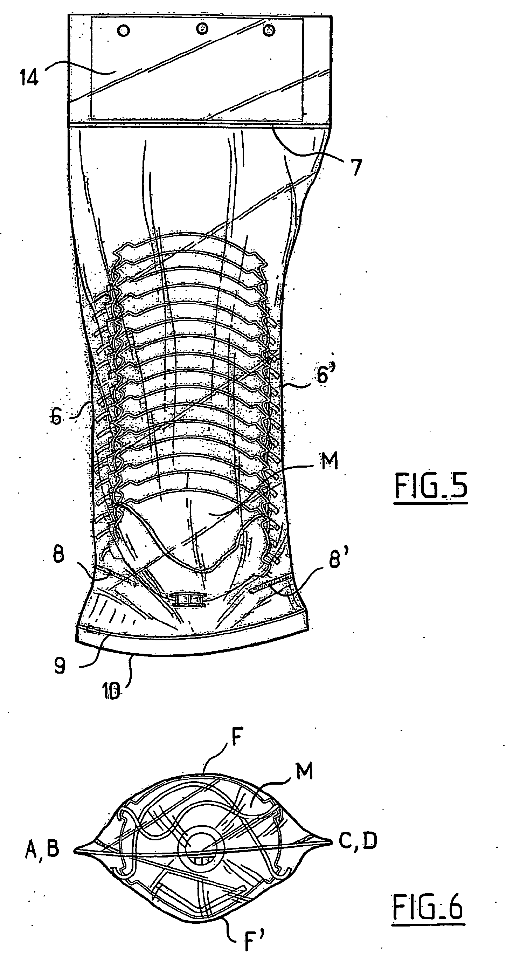

Les deux parois sont écartées l'une de l'autre, entre les soudures longitudinales et les soudures transversales, par la pile des masques (comme on le voit sur les figures 5 et 6). De fait la soudure (7) n'est réalisée qu'après introduction de la pile entre les deux parois.

L'autre soudure transversale (7') est réalisée à tout moment approprié pour sceller inférieurement la gaine ; cette dernière soudure est apte à être découpée pour la mise en service du distributeur. Une fermeture à curseur (9) est éventuellement réalisée entre la soudure (7') et les soudures obliques (8, 8') pour que la sortie (10) de la poche soit maintenue fermée à la demande après suppression de la soudure (7').

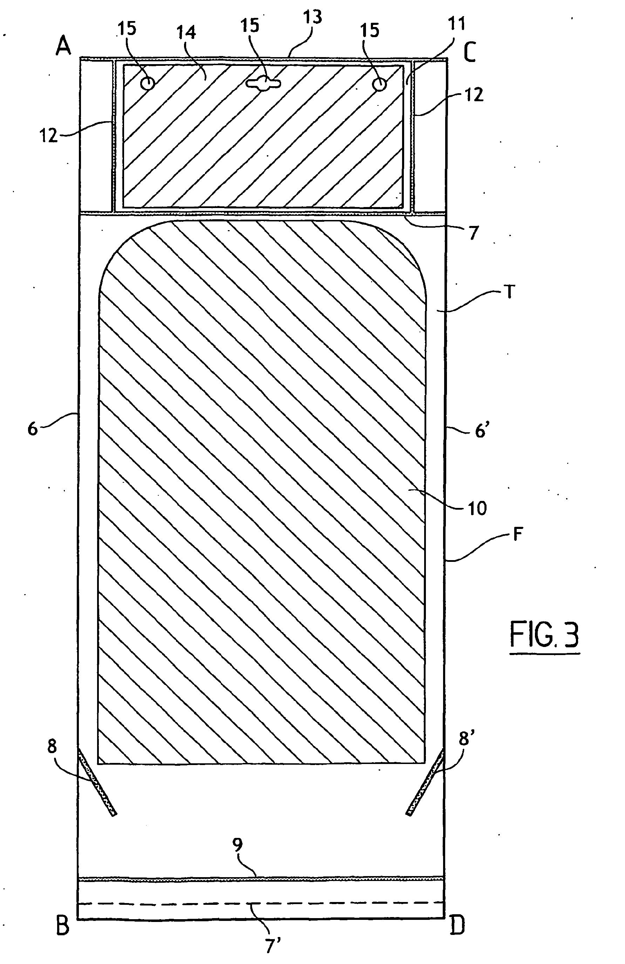

Avantageusement, comme on le voit sur la figure 3, une plaquette de renfort (10), par exemple un carton, est glissée dans la poche T pour rigidifier la poche qui par nature est relativement souple et déformable. Sur cette plaquette peuvent être imprimés des pictogrammes et toutes indications utiles ou publicitaires.

En partie supérieure, au dessus du soudage transversal (7), les deux parois de la gaine forment au dessus de la poche un logement plat (11) délimité par des lignes de soudage (12, 13), qui peut recevoir une plaquette (14) de rigidification (figure 3). La plaquette et les parois de la gaine sont perforées (15) pour permettre la suspension du distributeur.

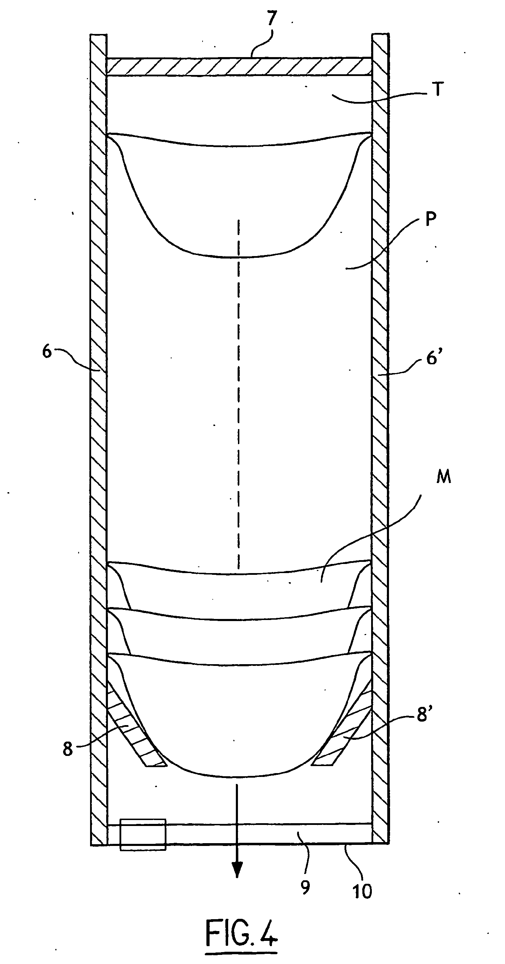

La figure 4 est limitée à la région de la gaine ou se trouve la pile (P) de masques (M). On y voit les lignes de soudage (6,6', 7, 8,8') et une fermeture à curseur (9) (éventuelle) de la sortie (10) de la poche (T).

Lorsque la sortie de la poche est à l'état ouvert, le masque inférieur de la pile peut être extrait par une traction manuelle.

L'invention n'est pas limitée à cet exemple de réalisation.

## Revendications

1. Distributeur pour distribuer des masques faciaux M empilés avec leurs collerettes (2) tournées vers le haut, ces masques étant déformables transversalement et conformés pour présenter un fond (3) et, à l'opposé de ce fond, une ouverture large bordée par une collerette (2), et apte à s'appliquer sur le visage par la collerette en couvrant la bouche et le nez, et entre le fond et la collerette une paroi latérale filtrante (1, 1') sans raideur, ce distributeur étant du type à suspendre, à sortie par le bas, apte à contenir à coulisse une pile verticale (P) des masques faciaux dans une chambre séparée de cette sortie par une zone où la section droite de la chambre est rétrécie afin d'arrêter le masque inférieur de la pile, tout en permettant d'extraire à force ce masque par la section rétrécie, ce distributeur étant **caractérisé en ce qu'**il est essentiellement constitué par une gaine tubulaire allongée, déformable et non extensible, en film mince thermosoudable, qui forme deux parois (F,F') en vis-à-vis solidarisées par leurs bords longitudinaux AB,CD et maintenues localement écartées l'une de l'autre en formant une poche par la pile des masques contenue dans la gaine, les deux parois étant soudées l'une à l'autre sous la pile par deux soudures obliques (8,8') en vis-à-vis qui partent des bords longitudinaux des deux parois en réduisant progressivement la section droite de la gaine jusqu'à une section rétrécie en sorte que le masque inférieur de la pile soit retenu entre les soudures obliques par contact latéral avec la gaine et que le fond de ce masque soit en saillie sous la section rétrécie, et la gaine présentant en dessous de la section rétrécie une sortie de gaine (10) située au dessous du fond du masque inférieur, et fermée au moins provisoirement par une fermeture (7', 9).

2. Distributeur selon la revendication 1 dans lequel ladite gaine est fermée au dessus de la pile par soudage transversal (7) des deux parois l'un contre l'autre.

3. Distributeur selon la revendication 2 dont les deux parois constituent au dessus du soudage transversal (7) un logement plat (11) apte à contenir une plaquette de rigidification (14).

4. Distributeur selon la revendication 3 dont ledit logement plat et la plaquette comportent des perforations (15) qui permettent d'accrocher le distributeur à un support.

5. Distributeur selon l'une des revendications 1 à 4 et qui contient entre la pile des masques et l'une des parois (F,F') une plaquette de rigidification (10).

6. Distributeur selon l'une des revendications 1 à 5 dont les deux parois (F,F') sont fixés l'une à l'autre par des soudures longitudinales (6,6').

7. Distributeur selon l'une des revendications 1 à 6, dont la gaine est scellée inférieurement par une soudure transversale (7') à déchirer pour la mise en service du distributeur.

8. Distributeur selon l'une des revendications 1 à 5, et dont la gaine est fermée inférieurement par une fermeture (9) à glissière.

9. Distributeur selon l'une des revendications 1 à 8 dont ledit film est transparent.

10. Distributeur selon la revendication 9 dont ledit film est en polyéthylène.

11. Distributeur selon l'une des revendications 1 à 10 dont les masques sont constituées par une coquille à double paroi (1,1') en non tissé qui emprisonne un média filtrant.

## Claims

1. Dispenser for dispensing face masks M stacked with their flanges (2) facing upwards, these masks being deformable transversely and configured to have a front end (3) and, on the opposite side to this front end, a wide opening which is bordered by a flange (2) and is able to be applied over the face by way of the flange so as to cover the mouth and the nose, and, between the front end and the flange, a filtering lateral wall (1, 1') without any stiffness, this dispenser being of the type which is to be suspended, has a bottom-dispensing outlet and is designed to contain, such that it can slide, a vertical stack (P) of face masks in a chamber separated from this outlet by a region in which the cross section of the chamber is narrowed so as to arrest the lower mask of the stack, while at the same time allowing this mask to be withdrawn by force through the narrowed section, this dispenser being **characterized in that** it is essentially composed of a deformable and non-extensible elongate tubular sleeve made of heat-weldable thin film, the sleeve forming two mutually opposite walls (F, F') which are secured by their longitudinal edges AB, CD and held locally apart from one another, forming a pocket, by the stack of masks contained in the sleeve, the two walls being welded to one another below the stack by means of two mutually opposite oblique welds (8, 8') which start from the longitudinal edges of the two walls, progressively reducing the cross section of the sleeve, and terminate at a narrowed section, such that the lower mask of the stack is retained between the oblique welds by lateral contact with the sleeve and such that the front end of this mask projects below the narrowed section, and the sleeve having, beneath the narrowed section, a sleeve outlet (10) situated beneath the front end of the lower mask and closed at least temporarily by a closure (7', 9).

2. Dispenser according to Claim 1, in which the said sleeve is closed above the stack by welding the two walls against one another with a transverse weld (7).

3. Dispenser according to Claim 2, wherein the two walls constitute, above the transverse weld (7), a flat housing (11) designed to contain a stiffening insert (14).

4. Dispenser according to Claim 3, wherein the said flat housing and the insert comprise perforations (15) by means of which the dispenser can be attached to a support.

5. Dispenser according to one of Claims 1 to 4, which contains a stiffening insert (10) between the stack of masks and one of the walls (F, F').

6. Dispenser according to one of Claims 1 to 5, wherein the two walls (F, F') are fastened to one another by longitudinal welds (6, 6').

7. Dispenser according to one of Claims 1 to 6, wherein the sleeve is sealed at its bottom by a transverse weld (7') which is to be torn off to make the dispenser operational.

8. Dispenser according to one of Claims 1 to 5, wherein the sleeve is closed at its bottom by a zip-type closure (9).

9. Dispenser according to one of Claims 1 to 8, wherein the said film is transparent.

10. Dispenser according to Claim 9, wherein the said film is made of polyethylene.

11. Dispenser according to one of Claims 1 to 10, wherein the masks are composed of a shell having a nonwoven double wall (1, 1') which entraps a filtering medium.

## Patentansprüche

1. Verteilautomat zum Ausgeben von Gesichtsmasken M, die mit ihren Randstreifen (2) nach oben zeigend gestapelt sind, wobei diese Masken in Querrichtung verformbar sind und dafür eingerichtet sind, einen Boden (3) und, diesem Boden gegenüberliegend, eine breite Öffnung, die von einem Randstreifen (2) eingefaßt ist, aufzuweisen, und sie dafür geeignet sind, mit dem Randstreifen auf das Gesicht aufgedrückt zu werden, wobei Mund und Nase bedeckt werden, und zwischen dem Boden und dem Randstreifen eine filtrierende Seitenwand (1, 1') ohne Steifigkeit aufzuweisen, wobei dieser Verteilautomat ein Typ zum Aufhängen mit dem Ausgang nach unten ist und er dafür geeignet ist, verschiebbar einen vertikalen Stapel (P) der Gesichtsmasken zu enthalten, und dies in einer Kammer, die von diesem Ausgang durch einen Bereich getrennt ist, in dem der Querschnitt der Kammer verengt ist, um die untere Maske des Stapels aufzuhalten und dabei gleichzeitig diese Maske durch Kraft durch den verengten Querschnitt herausnehmen zu können, wobei dieser Verteilautomat **dadurch gekennzeichnet ist, daß** er im wesentlichen durch einen langgestreckten röhrenförmigen Mantel gebildet wird, der verformbar und nicht streckbar ist und der aus einer dünnen wärmeverschweißbaren Folie besteht, die zwei sich gegenüberstehende Wände (F, F') bildet, die über ihre Längsränder AB, CD fest miteinander verbunden sind und die durch die im Mantel enthaltenen Masken lokal voneinander auf Abstand gehalten werden, wobei sie eine Tasche ausbilden und wobei die beiden Wände unterhalb des Stapels miteinander verschweißt sind durch zwei sich gegenüberliegende und schräg verlaufende Verschweißungen (8, 8'), die von den Längsrändern der beiden Wände ausgehen und dabei den Querschnitt des Mantels zunehmend verengen, bis zu einem derart verengten Querschnitt, daß die untere Maske des Stapels zwischen den schrägen Verschweißungen durch einen seitlichen Kontakt mit dem Mantel zurückgehalten wird und daß der Boden dieser Maske unterhalb des verengten Querschnitts hervorsteht, und wobei der Mantel unterhalb des verengten Querschnitts einen Mantelausgang (10) hat, der sich unterhalb des Bodens der unteren Maske befindet und der wenigstens provisorisch durch einen Verschluß (7', 9) verschlossen ist.

2. Verteilautomat nach Anspruch 1, bei dem der Mantel oberhalb des Stapels durch eine querverlaufende Verschweißung (7) der beiden Wände miteinander verschlossen ist.

3. Verteilautomat nach Anspruch 2, bei dem die beiden Wände oberhalb der querverlaufenden Verschweißung (7) eine flache Aufnahme (11) bilden, die dafür geeignet ist, eine Versteifungsplatte (14) aufzunehmen.

4. Verteilautomat nach Anspruch 3, bei dem die flache Aufnahme und die Platte Lochungen (15) umfassen, die es gestatten, den Verteilautomaten an einem Träger aufzuhängen.

5. Verteilautomat nach einem der Ansprüche 1 bis 4, der zwischen dem Maskenstapel und einer der Wände (F, F') eine Versteifungsplatte (10) enthält.

6. Verteilautomat nach einem der Ansprüche 1 bis 5, dessen beiden Wände (F, F') durch in Längsrichtung verlaufende Verschweißungen (6, 6') aneinander befestigt sind.

7. Verteilautomat nach einem der Ansprüche 1 bis 6, dessen Mantel unten durch eine querverlaufende Verschweißung (7') versiegelt ist, die für eine Inbetriebnahme des Verteilungsautomaten aufzureißen ist.

8. Verteilautomat nach einem der Ansprüche 1 bis 5, dessen Mantel unten durch einen Reißverschluß (9) verschlossen ist.

9. Verteilautomat nach einem der Ansprüche 1 bis 8, dessen Folie durchsichtig ist.

10. Verteilautomat nach Anspruch 9, dessen Film aus Polyethylen ist.

11. Verteilautomat nach einem der Ansprüche 1 bis 10, dessen Masken aus einer doppelwandigen und nicht gewebten Schale (1, 1') gebildet sind, die ein filtrierendes Medium einschließt.
